## Europäisches Patentamt
## European Patent Office
## Office européen des brevets

(19)

(11) Numéro de publication: **0 079 633**
**B1**

(12)
# FASCICULE DE BREVET EUROPÉEN

(45) Date de publication du fascicule du brevet:
**04.02.87**

(51) Int. Cl.⁴: **A 61 C 5/14,** A 61 C 17/04,
A 61 C 19/00

(21) Numéro de dépôt: **82201330.6**

(22) Date de dépôt: **26.10.82**

(54) Perfectionnement à un pare-langue paralléliseur fixe pour la médecine dentaire.

(30) Priorité: **06.11.81 BE 6047550**

(43) Date de publication de la demande:
**25.05.83 Bulletin 83/21**

(45) Mention de la délivrance du brevet:
**04.02.87 Bulletin 87/6**

(84) Etats contractants désignés:
**AT CH DE FR GB IT LI LU NL SE**

(56) Documents cité:
**EP-A-0 026 515**
**CH-A-164 668**
**DE-C-1 089 118**
**FR-A-2 347 918**
**US-A-2 382 385**
**US-A-2 510 077**
**US-A-3 805 389**

(73) Titulaire: **PROMODENT, Société anonyme, 21, rue Léon Mignon, B-4000 Liège (BE)**

(72) Inventeur: **Streel, Robert, 21, rue Léon Mignon, B-4000 Liège (BE)**

(74) Mandataire: **Dellicour, Paul, Office de Brevets E. Dellicour rue Fabry 18/012, B-4000 Liège (BE)**

EP 0 079 633 B1

## Description

Un pare-langue paralléliseur fixe, qui est un instrument de médecine dentaire destiné à permettre de dégager au maximum le champ opératoire et de faciliter la préparation et la pose de piliers postérieurs d'une prothèse fixe, comporte suivant EP-A-0 026 515 un moyen de fixation à la mandibule, dans sa partie antérieure sous forme de gouttière à remplir de matière plastique se solidifiant et un moyen de fixation à la mandibule dans sa région sousmentonnière réglable et verrouillable, les deux moyens de fixation étant montés sur un élément commun indépendant du tube d'aspiration du pare-langue proprement dit.

Dans ce pare-langue les deux moyens de fixation sont montés sur leur élément de support commun par des organes réglables et verrouillables tels que des vis.

Parmi les pare-langues connus US-A-2 382 385 décrit un instrument constitué par un ensemble comportant un pare-langue proprement dit, un moyen de fixation à la mandibule dans sa partie antérieure et un moyen de fixation à la mandibule dans la région sous-mentonnière réglable en hauteur et verrouillable, ces moyens étant reliés par un ensemble de deux tiges accolées latéralement, sur lesquelles la mentonnière se bloque automatiquement à la hauteur souhaitée.

On connaît aussi, par DE-C-1 089 118, un pare-langue, dont la mentonnière est réglable en hauteur le long d'un tube, associé à une tige destinée à empêcher un pivotement, et bloquée à la hauteur souhaitée par un dispositif de serrage tel qu'une vis.

Suivant le présent perfectionnement il a paru avantageux d'apporter à un pare-langue paralléliseur fixe du type décrit ci-dessus quelques modifications pour améliorer le positionnement de l'instrument, éviter toute rotation d'une pièce par rapport à l'autre et faciliter le travail en bouche.

Un pare-langue paralléliseur fixe suivant le présent perfectionnement du type de l'appareil décrit dans EP-A-0 026 515, comportant un pare-langue proprement dit, formé par une plaque verticale avec drainage de la salive, une gouttière à remplir de matière plastique qui se solidifie, destinée à fixer l'instrument à la partie antérieure de la mandibule, une mentonnière réglable et verrouillable, destinée à fixer l'instrument à la région sous-mentonnière de la mandibule, et un élément de support, appelé valet, reliant la gouttière à la mentonnière, est caractérisé en ce que le valet est en forme de F dont les deux branches sont disposées sagittalement, en ce que la mentonnière se bloque automatiquement sur les deux branches du valet et consiste en une plaque d'assise ajourée en son centre et qui se prolonge par une branche de support pourvue d'une série de perforations formant glissières pour les branches du valet, en ce que la gouttière fait partie intégrante du valet et est prévue à l'extrémité de la branche du valet, à l'opposé des branches sagittales et en ce que la plaque du pare-langue se prolonge par une branche de support qui le relie à la branche du valet par un dispositif de blocage, qui comprend un levier à came manoeuvrable dans un plan vertical en dehors de la bouche.

Egalement suivant le perfectionnement, sur le pare-langue proprement dit en forme de plaque verticale est monté un écarteur de joue constitué par un élément élastique tel qu'un ressort.

Le perfectionnement sera mieux compris grâce à la description qui suit, basée sur le dessin annexé à titre d'exemple et montrant en :

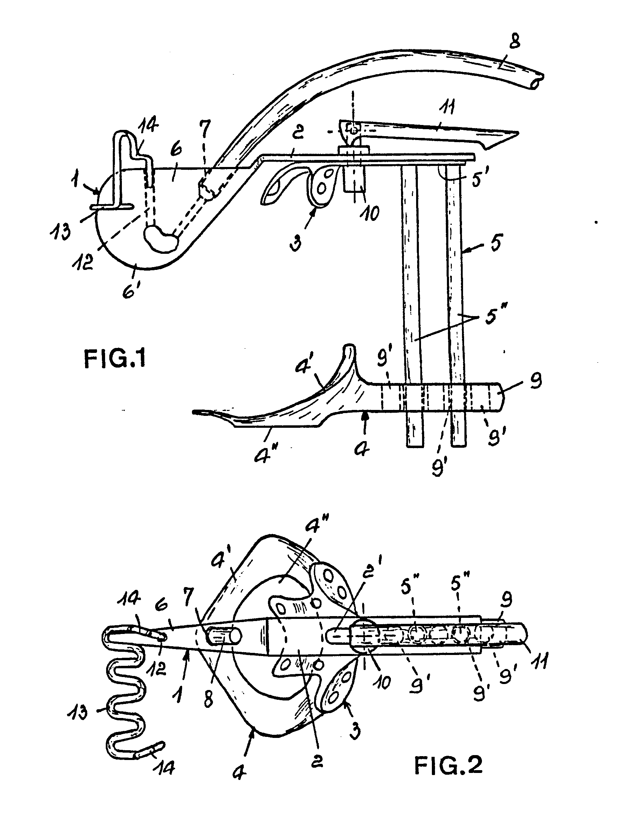

Figure 1 une vue en élévation du pare-langue paralléliseur fixe perfectionné, et

Figure 2 une vue en plan par dessus du pare-langue de la figure 1.

On voit au dessin l'instrument se composant du pare-langue proprement dit 1 avec son support 2, un dispositif de fixation à l'arcade inférieure conformé en forme de gouttière 3 à remplir de matière plastique se solidifiant, un dispositif de fixation sous mentonnier 4 est un organe d'assemblage et de solidarisation 5.

Le pare-langue proprement dit 1 est constitué comme dans EP-A-0 026 515. On retrouve la plaque verticale 6 et sa partie recourbée 6', l'embout 7 et le raccord flexible 8 communiquant avec la pompe d'aspiration courante. Le pare-langue proprement dit se prolonge par une branche de support 2 le reliant à l'ensemble formé par le moyen de fixation à la mandibule dans sa partie antérieure ou gouttière 3 et le moyen de fixation à la mandibule dans la région sous-mentonnière ou mentonnière 4, reliés entre eux par une pièce 5 en forme de F constituant un valet à serrage automatique.

La gouttière 3 est soudée à l'extrémité de la branche 5' de la pièce 5 posée de chant à l'opposé des branches parallèles 5" disposées sagittalement, et la mentonnière est constituée par une plaque d'assise 4' se prolongeant par une branche 9 d'une certaine épaisseur munie d'une série de perforations 9'. La plaque 4' constituant la mentonnière proprement dite permet un appui étalé d'une seule pièce. Elle est constituée en forme et présente au centre un ajourage 4" réalisant un maintien par les tissus mous de la partie externe du menton.

Les branches parallèles sagittales 5" du valet 5, formées de tiges rondes, sont enfilées dans deux des perforations 9' de la branche 9, perforations qui constituent des coulisses ou glissières parallèles de manière à permettre un positionnement à des distances différentes et ainsi un ajustement en bonne position suivant le type de mâchoire et la situation du menton.

Le blocage en hauteur de la mentonnière 4 est réalisé grâce à l'effet de valet qui cale sa branche 9 par suite de la pression contre l'axe des deux tiges verticales 5". Ceci remplace avantageusement tout dispositif de fixation en hauteur à molette.

La mentonnière 4 et le dispositif de fixation à l'arcade 3 sont solidaires dans un plan et leur

assemblage ne permet pas de rotation de l'un par rapport à l'autre.

La liaison entre l'ensemble 3, 4, 5 et la branche support 2 du pare-langue proprement dit 1 est réalisée par une broche 10 portée par la branche 5' du valet, en avant de la gouttière 3, et traversant une coulisse 2' prévue dans la branche support 2. Cette broche sert d'axe à un levier à came 11 destiné au blocage, dans la position longitudinale et angulaire souhaitée, de la branche 2 sur la branche 5' c'est-à-dire du pare-langue proprement dit. Ce levier à came 11 est agencé de manière à pouvoir se déplacer dans le plan vertical en dehors de la bouche.

Comme déjà prévu la plaque 6 du pare-langue proprement dit 1 présente un logement vertical 12 servant au placement d'une tige de paralléliseur. Suivant le présent perfectionnement, ce logement 12, qui peut servir d'ancrage à tous systèmes destinés à transférer l'axe des dents à distance de l'arcade antérieure et à constituer un support éventuel pour une pièce de liaison avec tout instrument pour travailler dans une direction déterminée ou pour un support de stabilisation horizontale, permet l'ajustage d'une pièce additionnelle conformée pour servir d'écarteur de joue 13.

Cet écarteur de joue, servant à mieux dégager encore le champ opératoire, est constitué par un élément élastique tel qu'un ressort qui se place en saillie de l'un ou l'autre côté de la plaque 6, à laquelle il s'accroche par son extrémité correspondante conformée en forme de crochet 14 venant s'insérer dans le logement 12. Suivant le côté de la bouche qui doit être libre et bien dégagé, l'écarteur de joue 13 est placé à gauche du pare-langue proprement dit, comme au dessin, ou à droite.

Un pare-langue tel que décrit ci-dessus, qui ne blesse pas du fait qu'il est fixé à l'arcade par la pâte solidifiée, peut en dégageant au maximum le champ opératoire être déplacé latéralement et en profondeur de manière simple, rapide et sûre.

## Revendications

1. Pare-langue paralléliseur fixe comportant un pare-langue (1) proprement dit, formé par une plaque verticale (6) avec drainage (8) de la salive, une gouttière (3) à remplir de matière plastique qui se solidifie, destinée à fixer l'instrument à la partie antérieure de la mandibule, une mentonnière (4) réglable et verrouillable, destinée à fixer l'instrument à la région sous-mentonnière de la mandibule, et un élément de support (5), appelé valet, reliant la gouttière (3) à la mentonnière (4), caractérisé en ce que le valet (5) est en forme de F dont les deux branches (5'') sont disposées sagittalement, en ce que la mentonnière se bloque automatiquement sur les deux branches (5'') du valet et consiste en une plaque d'assise ajourée en son centre et qui se prolonge par une branche de support (9) pourvue d'une série de perforations (9') formant glissières pour les branches (5'') du valet, en ce que la gouttière (3) fait partie intégrante du valet (5) et est prévue à l'extrémité de la branche (5') du valet, à l'opposé des branches sagittales (5''), et en ce que la plaque (6) du pare-langue (1) se prolonge par une branche de support (2) qui le relie à la branche (5') du valet par un dispositif de blocage, qui comprend un levier à came (11) manoeuvrable dans un plan vertical en dehors de la bouche.

2. Pare-langue suivant la revendication 1, caractérisé en ce que dans la plaque (6), constituant le pare-langue proprement dit, est prévu un logement (12) pour l'organe de support d'un élément (13) formant écarteur de joue.

3. Pare-langue suivant la revendication 2, caractérisé en ce que l'écarteur de joue (13) est constitué d'un élément élastique tel qu'un ressort, dont les extrémités (14) sont conformées en forme de crochet destiné à venir s'insérer dans le logement (12) de la plaque verticale (6) pour un positionnement en saillie de l'un ou l'autre côté par rapport à ladite plaque.

## Patentansprüche

1. Zungenschutz mit festem Parallelanzeiger bestehend aus genau genommen einem Zungenschutz (1), der aus einer vertikalen Platte (6) gebildet ist, mit einer Speichelabsaugung (8), einer Einfüllrinne (3) zum Einfüllen von Plastikmaterial, das sich verfestigt, wobei dieser dazu vorgesehen ist, die Vorrichtung im Vorderbereich des Unterkiefers zu befestigen, eine verstellbare und verriegelbare Kinnstütze (4), die dazu vorgesehen ist, die Vorrichtung im Unterkinnbereich des Kiefers zu fixieren, und ein Trägerelement (5), genannt Klemmstange, das die Rinne (3) und die Kinnstütze (4) verbindet, dadurch gekennzeichnet, daß die Klemmstange (5) in Form eines F ausgebildet ist, dessen beide Arme (5'') pfeilförmig angeordnet sind, so daß sich die Kinnstütze automatisch zwischen den beiden Armen (5'') der Klemmstange einklemmt und aus einer Grundplatte besteht, welche in ihrem Mittelteil durchbrochen ist und die sich in einem Tragarm (9) fortsetzt, der mit einer Reihe von Ausnehmungen (9'), die Führungen für die Arme (5'') der Klemmstange bilden, versehen ist, daß die Rinne (3) ein festintegriertes Teil der Klemmstange (5) ist und am äußeren Ende des Armes (5') der Klemmstange gegenüber den pfeilförmigen Armen (5'') ausgebildet ist und daß die Platte (6) des Zungenschutzes (1) mit einem Tragarm (2) verlängert ist, der mit dem Arm (5') der Klemmstange mittels einer Blockiereinrichtung verbunden ist, die einen Feststellhebel (11) umfaßt, welcher in einer vertikalen Ebene außerhalb des Mundes bedienbar ist.

2. Zungenschutz gemäß Anspruch 1,

dadurch gekennzeichnet,
daß in der Platte (6), die genauer gesagt den Zungenschutz bildet, eine Ausnehmung (12) zur Aufnahme für ein Trägerelement für ein Element (13), welches eine Spreizvorrichtung für die Backen bildet, vorgesehen ist.

3. Zungenschutz gemäß Anspruch 2,
dadurch gekennzeichnet,
daß die Spreizvorrichtung für die Backen (13) aus einem elastischen Element, zum Beispiel einer Feder, besteht, dessen äußere Teile (14) in Form eines Hakens geformt sind, der dazu bestimmt ist, aus der Ausnehmung (12) der vertikalen Platte (6) herauszuragen, um eine Lage außerhalb der einen oder anderen Seite bezüglich der obengenannten Platte einzunehmen.

**Claims**

1. Fixed paralleliser tongue guard comprising a tongue-guard proper (1) consisting of a vertical plate (6) with a saliva drain (8), a channel (3) to be filled with plastics material which solidifies, intended to fix the instrument to the front part of the jawbone, an adjustable and lockable chinrest (4) intended for fixing the instrument to the under-chin area of the jawbone, and a support element (5), or rest, connecting the channel (3) to the chinrest (4), characterized in that the rest (5) is F-shaped, its two arms (5") being disposed sagitally in that the chinrest is automatically locked on the two arms (5") of the rest (5) and consists of a seating plate with an opening in its centre and which extends by a support arm (9) provided with a series of apertures (9') which form slideways for the arms (5") of the rest (5) and in that the channel (3) forms an integral part of the rest (5) and is provided at the end of the arm (5') of the rest (5), opposite the sagittal arms (5") and in that the plate (6) of the tongue guard (1) is extended by a support arm (2) which connects it to the arm (5') of the rest (5) via a locking device which comprises a cam lever (11) manoeuvrable in a vertical plane outside the mouth.

2. Tongue-guard according to Claim 1, characterized in that there is in the plate (6) constituting the actual tongue-guard a housing (12) for the member which supports an element (13) which constitutes a cheek separator.

3. Tongue-guard according to Claim 2, characterized in that the cheek separator (13) consists of a resilient element such as a spring, of which the ends (14) are shaped like a hook adapted to be inserted into the housing (12) in the vertical plate (6) for a positioning to project from one side or the other in relation to the said plate.

FIG.1

FIG.2